# EUROPEAN PATENT APPLICATION

(11) **EP 2 390 809 A1**
(43) Date of publication of application: **30.11.2011**
(21) Application number: 11168169.8
(22) Date of filing: 31.05.2011
(51) Int. Cl.: G06F 19/00

(54) **System and method for managing health data**

(30) Priority: 31.05.2010 IT MI20100983
(71) Applicant: WSC World System Consulting S.r.l., 10036 Settimo Torinese (IT)
(72) Inventor: Tarulli, Olivia, 10051 AVIGLIANA (IT); Malerba, Damiano, 10100 TORINO (IT); Altobelli, Giuseppe, 10051 AVIGLIANA (IT)
(74) Representative: De Ros, Alberto

(57) **Abstract**

A system and method for managing health data based on a reader of a storage unit containing at least one health datum, said reader comprising an RFID tag reader containing a user code. The reader is configured to request, from a remote server by a local PC, validation of said user code and means for requesting entering of a password by said PC, and means for verifying said password, when said remote server is not accessible. When said user code or said password has been validated, the reader has access to said storage unit for reading/writing.

## Description

### Field of the invention

The present invention relates to a system and method for managing health data.

### Prior art

The hospital and health information and documentation of an individual's life is currently made available in paper form, with all the problems associated with managing the archiving of these documents and the respective retrieval when necessary.

The dynamic nature of modern life can mean that a person may be a long way from home, or from the location where said documentation is stored, sometimes for long periods.

It has already been envisaged for some time that this documentation should be digitized by production of documents directly in electronic format or by subsequent scanning.

It is clear that documents in computer format can be carried on oneself more easily, in view of the proliferation of mass storage devices that are extremely compact and have large storage capacities.

Numerous methods of data encryption can be used for protecting the data.

There are also mass storage devices whose file system is only accessible by password.

On the other hand, however, if a person is unable to supply the password, for example because they are unconscious, this memory device would not be usable by the hospital staff.

### Summary of the invention

The purpose of the present invention is to provide a method of managing health data which is able to solve the aforementioned problem.

The present invention relates to a method of managing health data according to claim 1.

The present invention also relates to a system for managing health data that is able to solve the aforementioned problem.

The present invention relates to a system for managing health data according to claim 7.

Advantageously, the present invention makes it possible to obtain
- greater security of access and storage of data in a portable storage unit,
- management of data access of the multiuser and multilevel type, by certification of the user and definition of the associated rights of access to the data,
- simplification of storage-acquisition of health data.

Another purpose of the present invention is to provide a reader of storage units containing health data that contributes to solving the aforementioned problem. The present invention also relates to a storage unit reader according to claim 9. Hereinafter, "individual" means a possessor of a storage unit, in which the relevant health data are contained; moreover, "user" means someone who intends to access said data either for reading or for writing.

The dependent claims describe preferred embodiments of the invention forming an integral part of the present description.

### Brief description of the drawings

Further characteristics and advantages of the invention will become clearer from the detailed description of preferred, but not exclusive, embodiments of a system and method for managing health data, illustrated with non-limiting examples, with the aid of the accompanying drawings in which:

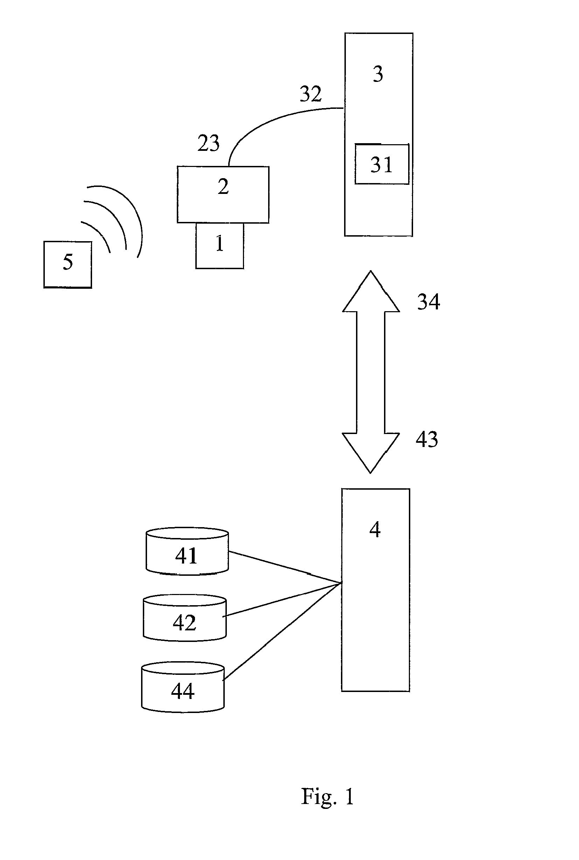

Fig. 1 is a schematic representation of an example of a system according to the present invention;

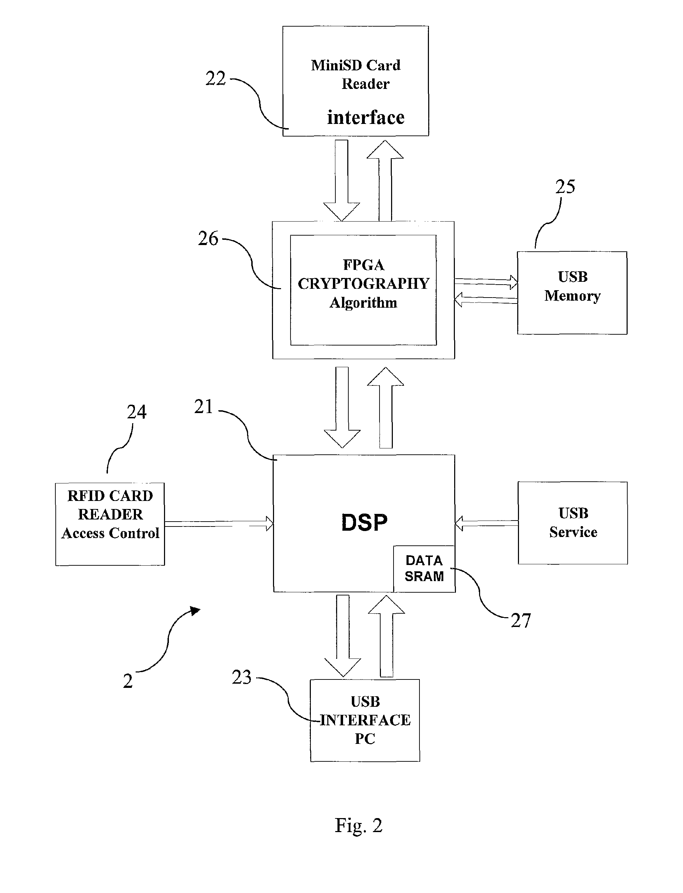

Fig. 2 shows a logic diagram of a part of the system of Fig. 1;

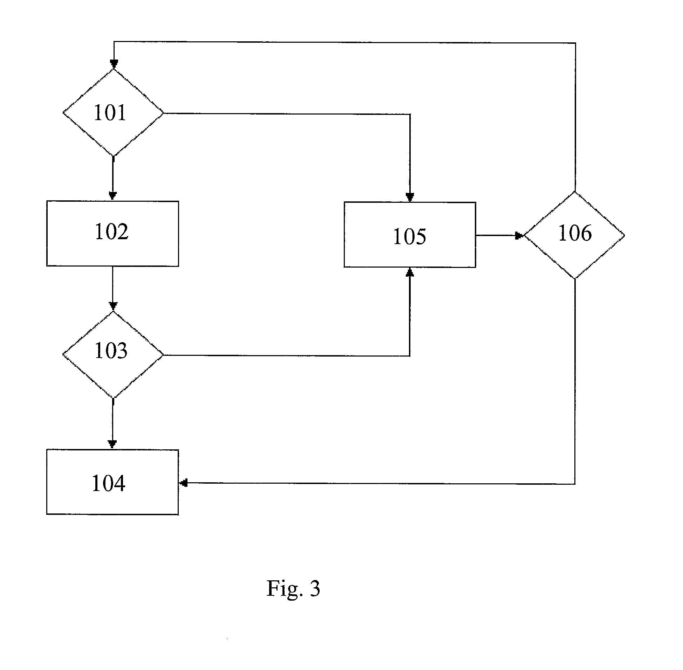

Fig. 3 shows a flow chart of the operation of the system according to Fig. 1;
Fig. 4 shows a detailed flow chart of an operating mode of the system of Fig. 1;
Fig. 5 shows a detailed flow chart of another operating mode of the system of Fig. 1.

The same reference numbers and letters in the figures identify the same elements or components.

### Detailed description of a preferred embodiment of the invention

According to a preferred embodiment of the invention, a preferred system comprises:
- a portable storage unit 1 comprising
   ○ a free access partition in which there are free access files, for example an individual's personal information file and files necessary for installation of one or more software applications 31 intended to run on a PC, in such a way that said data are accessible without authentication, and
   ○ at least one protected partition, inaccessible without authentication or encrypted,
- Said storage unit 1 is intended to be entrusted to an individual and is intended to receive, in said protected partition, the individual's health data, for example results of analyses, prescriptions, diagnoses, radiological examinations, etc.,
- a reader 2 of said storage unit 1 comprising a processing unit 21 containing and executing firmware 21', a first interface 22 compatible with said storage unit 1 and connected to said processing unit 21, a second interface 23 connected to said processing unit 21 and able to interface with a PC, a third interface RFID tag reader 24 connected to said processing unit 21 and able to read a user code from an RFID tag 5 of a user who is about to access said storage unit; said processing unit 21 is configured so that said reader 2, when connected to a PC, is recognized as a hub for removable storage units and comprises at least one storage area of its own 27 which stores applications that are executable automatically by the PC (Autorun) at the moment of connecting reader 2 to the PC;
- a PC 3 connected to said reader 2; the computer comprises an interface 32 with said reader 2 and a remote connection 34 to a remote server 4,
- a remote server 4 for authenticating a user code comprising a remote connection 43 to said PC 3,
- an RFID tag 5 containing a user code relating to a user.

Thus, according to the present invention, the installation files of a software application 31 that runs on said PC 3 are stored beforehand in said storage unit 1, in the free partition, and/or in said storage area of said reader 2.

Said user is generally a health worker, typically a doctor or a nurse, and said individual is typically a patient.

Said second interface 23 can be either of the wireless type (WiFi, Bluetooth, etc.) or of the cabled type (USB, Ethernet, Parallel, Firewire, etc.).

Thus, said reader 2 permits interfacing a PC, preferably local, with said storage unit 1. Moreover, said reader 2 comprises said RFID tag reader 24 for reading an identification code of a user who is about to access said storage unit. In particular, authentication of the user is transferred to said remote server 4. The reader 2, in fact, opens a connection, preferably in VPN, to the remote server 4 via said PC 3 and sends a request message containing the user code read by said RFID tag reader 24.

The remote server 4, comprising at least one first database 41 of users, verifies the user's credentials and sends a reply message containing instructions to the PC 3.

Said remote server 4, moreover, comprises a second database 42 of firmware updates of reader 2 of storage unit 1 and at least one third database 44 for backup of the health data contained in said storage unit 1.

It is clear from the architecture described that the health data contained in storage unit 1 are only made accessible after authentication of the user by the remote server 4.

According to another aspect of the present invention, accessibility to the data is also permitted in off-line mode by a suitable password in the individual's possession.

In this respect, the firmware 21' detects the availability of said remote connection 34/43 and the availability of said user code. When said connection is not available or when said user code is not available, said firmware opens a dialogue window in the man-machine interface provided by said PC 3, requesting the user to insert the password of the individual who owns the storage unit 1.

When the remote server 4 receives a request message for authentication, it verifies the existence of a corresponding user code in its own first database 41 and replies by sending a message stating the type of user and the permissions for reading/writing of storage unit 1. Conversely, if the user is not authenticated, then said message contains a refusal of authentication, therefore said firmware 21', as in the case of lack of availability of connection, asks for the individual's password to be entered.

Just as with the SIMs of cell phones, storage unit 1 can be blocked temporarily or permanently if a predefined number of incorrect entries of the password is exceeded.

When the remote connection is available and the user is correctly authenticated, the remote server can further verify the updating status of the firmware 21' of reader 2 and, if necessary, command a firmware updating procedure. Moreover, the remote server 4 can verify correspondence between the health data stored in storage unit 1 and the backup data stored in said third database 44, starting a procedure, preferably of the incremental type, for backup of the health data.

The server can, moreover, verify the integrity of the data and/or of the file system of storage unit 1, trying, if necessary, to restore them when corrupted or indicating what has occurred, so that the individual obtains a new storage unit 1.

With particular reference to the storage unit 1, it is at least logically partitioned into separate areas of memory in which data with lesser or greater confidentiality are stored.

A reader 2 connected to a PC 3 is recognized by the latter as a hub unit, in an area of internal storage, the drivers are present that are necessary for interfacing with the PC as well as for the files and programs necessary for initial installation of reader 2. Preferably, when the user has been authenticated and when said storage unit 1 is connected to reader 2, said firmware 21' executes instructions to said software application 31, previously installed on the PC 3, so that the latter produces a graphical interface containing a list of labels relating to selected health documents in relation to the user's credentials. Each element of the list also defines a connection to the relevant document so that it can be selected for display by the user.

Said software application 31 can advantageously be written in Microsoft.Net®, Java® and other languages.

This solution, advantageously, does not allow the user to access the data contained in the storage unit directly, instead access is mediated by the reader, which generates a dynamic view of the documents that can be displayed from time to time.

According to further variants of the system, software can be envisaged that can be installed in PC 3 for executing what has been described with respect to the firmware 21' and optionally for managing the operations of encryption/decryption of the documents written/read in said storage unit.

According to another aspect of the invention, generation of files, for example in HTML format, makes it possible to disengage the functionalities of reader 2 from the operating platform installed in the PC 3.

According to another preferred embodiment of the invention, instead, access to the health documents is controlled at file system level of storage unit 1, defining rights for reading and writing of the individual directories.

It is preferable, moreover, for data encryption to be of the hardware type, for example envisaging a suitable module 26 for encryption/decryption interposed between said processing unit 21 and said first interface 22 to the storage unit.

In order to prevent any data loss, it is preferable for said processing unit 21 to inhibit any operation of deletion of files or modification of files stored in storage unit 1.

A method of using the system therefore comprises the following phases:
- reader 2 with the relevant software 31 that runs on the PC 3 is waiting for reading of an RFID tag 5 to obtain a user code,
- when a user code has been obtained, a VPN connection is opened with said remote server 4, which verifies and recognizes the user code obtained and if in the affirmative enables the processing unit 21 with the relevant first interface 22;
- when a first storage unit 1 is connected to said first interface 22, the remote server 4 is asked to validate said first storage unit 1 and if in the affirmative
- said software 31 produces a view containing files with any descriptions relating to health information of the individual who owns the first storage unit,
- if instead a connection to the remote server 4 is not available or a user code has not been obtained, said software requests entering a password, if a valid password is entered then
- said software 31 attempts to access the data contained in the storage unit and, if the storage unit is compatible with the system, produces a view containing files with any descriptions relating to the health information of the individual who owns the first storage unit.

When a remote connection is available and both a user code and a storage unit have been validated, the remote server proceeds to synchronize/restore the data between said first storage unit and said third database 44.

In more detail, a preferred method comprises the following steps:
the method comprising the following steps:
   - (step 101) verification of the presence of a connection between said PC 3 and said remote server 4,
   - if said connection is present (step 2), reading of an RFID tag and verification (step 103) by said remote server 4 of a user code contained in said RFID tag,
   - if said user code is valid, enabling (step 104) of reader 2 for reading, and reading of a storage unit 1,
   - if instead a connection is not present between said PC 3 and said remote server 4 or if said user code is not verified, then (step 105) request for entering a password for enabling of reader (2) by said PC 3 and reading of a password (step 105) entered by said PC 3 and
   - (step 106) verification by said reader 2 of said password and, if said password is correct (step 104), enabling of reader 2 for reading said storage unit 1, otherwise it resumes from the beginning (step 101).

Said reader 2 can further comprise a third interface 25 for connection of devices for acquisition of images and documents in any format, such as scanners, TAC (TC), RM (MR), etc.

The documents and data that can be stored in said storage unit can be in any format, for example RTF, DOC, DOCX, PDF, IMG, BMP, PNG, DICOM, etc.

The free partition of storage unit 1 and/or the memory area of reader 27 can contain software applications for displaying particular formats of health data. Alternatively, connection to said devices can be performed by a further communication port or acquisition card of the PC to be addressed, under the control of said software 31, to the storage in said first storage unit 1.

The elements and the characteristics illustrated in the various preferred embodiments can be combined, while remaining within the scope of protection of the present application.

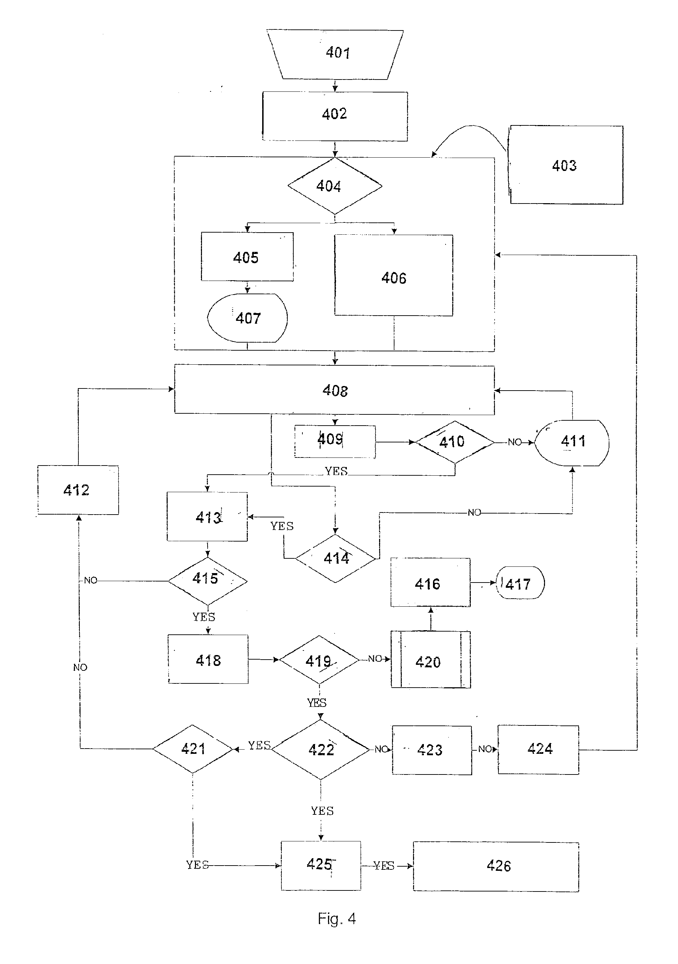

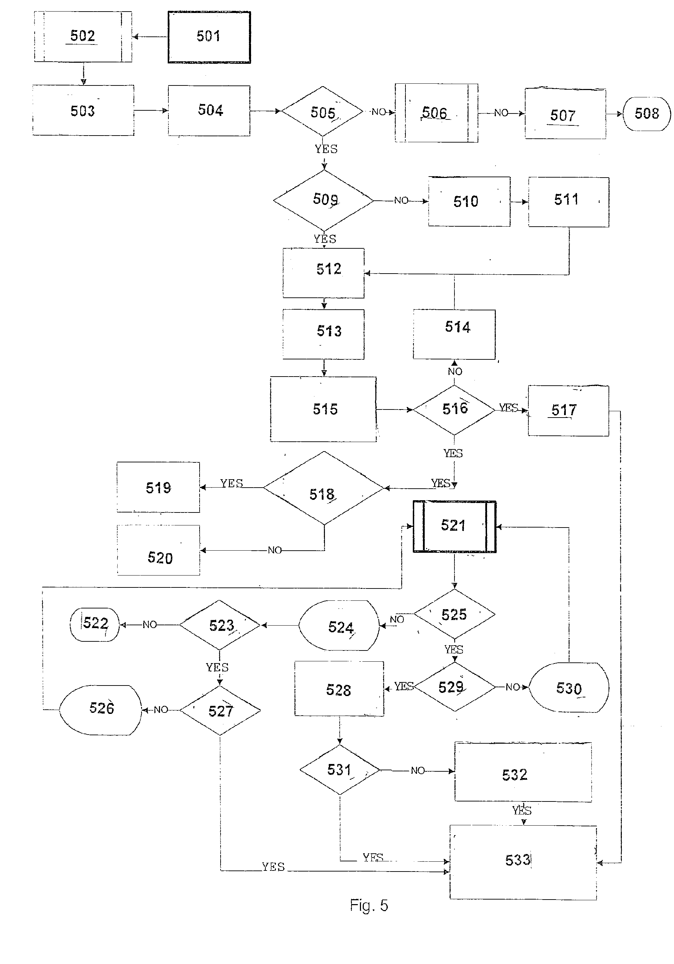

In the following tables there are the legends for the blocks of the flow charts of figures 4 and 5 with the correspondences between reference signs and block descriptions.

### LEGEND FIGURE 4

| **NUMBER** | **DESCRIPTION** |
|---|---|
| 401 | Connection of the Reader to the PC |
| 402 | The Reader controller senses the connection to the PC and starts the READER security software |
| 403 | Selection of the kind of activation of the Reader: through READER SECURITY PIN or through Medical authentication card |
| 404 | Selection of the type of activation |
| 405 | RFID reader ready waiting for reading TAG in the detection area |
| 406 | On the screen of the PC there is displayed the screenshot requesting security PIN (doctor/clinic) and the indication to slide the RFID card |
| 407 | The LED on the Reader is RED |
| 408 | The Reader keeps waiting |
| 409 | Input PSW |
| 410 | Valid PSW ? |
| 411 | Signalling error on the screen |
| 412 | Error message |
| 413 | Opening VPN Channel |
| 414 | Reading TAG |
| 415 | VPN Active ? |
| 416 | Message to remote centre security team |
| 417 | STOP |
| 418 | Checking READER number |
| 419 | Valid number ? |
| 420 | Sending message of READER not-authorized |
| 421 | Valid TAG ? |
| 422 | Firmware version up-to-date ? |
| 423 | START firmware update process |
| 424 | READER Reboot |
| 425 | Reader ACTIVE |
| 426 | Reader ready for reading patient cards |

### LEGEND FIGURE 5

| **NUMBER** | **DESCRIPTION** |
|---|---|
| 501 | START with reader ready |
| 502 | Request of authorization through doctor RFID CARD |
| 503 | ACTION opening PIN channel |
| 504 | Checking READER number |
| 505 | Valid number ? |
| 506 | Sending message of READER not-authorized |
| 507 | Message to remote centre security team |
| 508 | STOP |
| 509 | Firmware version up-to-date ? |
| 510 | START firmware update process |
| 511 | READER Reboot |
| 512 | READER waiting for activation |
| 513 | ACTION sliding Doctor CARD |
| 514 | Access Denied |
| 515 | Checking AUTHENTICATED ELECTRONIC SIGNATURE asymmetric key cryptography |
| 516 | Valid signature ? |
| 517 | Sending to the READER the access level of the doctor |
| 518 | Credentials valid for use of scanner ? |
| 519 | Opening scanner channel |
| 520 | Access Denied |
| 521 | Introducing Patient Memory Card |
| 522 | STOP |
| 523 | Inputting patient PSW ? |
| 524 | Error signalling |
| 525 | VPN active ? |
| 526 | Error signalling |
| 527 | Valid PSW ? |
| 528 | ACTION: checking versions of documents in patient card |
| 529 | Valid memory ? |
| 530 | Error signalling |
| 531 | Documents authorized ? |
| 532 | Start Synchronization process user backup area to operation center server |
| 533 | START software for visualization of patient card content based on level of doctor credentials |

## Claims

1. A method of managing health data by means of a system comprising
- a portable storage unit (1) comprising at least one health datum protected by a password,
- a reader (2) of said storage unit (1) comprising an RFID TAG reader (24) and processing means (21),
- a PC (3) connected to said reader (2) and comprising a first remote connection interface (34),
- a remote server (4) for authenticating a user code comprising a second remote connection interface (43),
- an RFID TAG (5) containing a user code related to a user,
the method comprising the following steps:
- (step 101) verifying the existence of a connection between said PC (3) and said remote server (4),
- if said connection exists (step 102) reading an RFID TAG and verifying (step 103) a user code contained in said RFID TAG by said remote server (4),
- if said user code is valid, enabling (step 4) the reader (2) to read and reading a storage unit (1),
- instead, if there is no connection between said PC (3) and said remote server (4) or if said user code is not verified, then (step 105) requesting to enter a password to enable the reader (2) by means of said PC (3), and reading a password (step 105) entered by said PC (3), and
- (step 106) verifying said password by means of said reader (2) and if said password is valid (step 104) enabling the reader (2) to read said storage unit (1), otherwise resuming the procedure from the beginning (step 101).

2. A method according to claim 1, wherein when said reader (2) is enabled to read a storage unit (1) and a storage unit (1) is connected to said reader (2), said processing means (21) carry out a step of making said health datum accessible in relation to authentication credentials of said user code.

3. A method according to claim 2, wherein said processing means carry out a step of generating a document containing an ordered list containing at least one label related to said datum and a hyperlink to said datum.

4. A method according to claim 1, comprising a step of storing a health datum in said storage unit (1).

5. A method according to claim 2 or 3 or 4, wherein access to a health datum in said storage unit (1) comprises an operation of decrypting said health datum, or an operation of storing a health datum comprises an operation of encrypting said health datum.

6. A method according to anyone of the preceding claims, wherein when said connection between said PC (3) and said remote server (4) is available, said remote server (4) carries out the following steps:
- verifying a firmware version installed on said reader (2),
- if said firmware version is not updated, updating the reader (2),
and/or the following steps:
- verifying a correspondence between said health datum contained in said storage unit (1) and between a backup contained in a remote database (44) and if no correspondence exists,
- starting a backup operation of said storage unit (1) on said remote database (44).

7. A system of managing health data comprising
- a portable storage unit (1) comprising at least one health datum protected by a password,
- a reader (2) of said storage unit (1) comprising an RFID TAG reader (24) and processing means (21),
- a PC (3) connected to said reader (2) and comprising a first remote connection interface (34),
- a remote server (4) for authenticating a user code comprising a second remote connection interface (43), comprising means for verifying a user code and determining credentials of a related user and means for sending a message containing said credentials,
- an RFID TAG (5) containing a user code related to a user,
wherein said processing means (21) are configured to:
- verify the presence of a connection between said PC (3) and said remote server (4),
- read an RFID TAG (5) when said connection exists and an RFID TAG (5) exists,
- send a request to validate a user code contained in said RFID TAG (5) to said remote server (4) and wait for said request being successful,
- wait for entering a password when said connection does not exist or when said validation request is not successful, and verify a validity of said password,
- read and write said storage unit (1) when said validation is positive or when said password is valid.

8. A system according to claim 7, wherein said processing means (21) are further configured to read said message sent by said remote server (4) and make said health datum accessible in relation to user's credentials contained therein.

9. A reader (2) of a removable storage unit (1) containing at least one protected health datum, the reader comprising a processing unit (21) containing and running a firmware (21'), a first interface (22) compatible with said storage unit (1) and connected to said processing unit (21), a second interface (23) connected to said processing unit (21) and adapted to interface with a PC (3), a third interface to an RFID TAG reader (24) connected to said processing unit (21) and adapted to read a user code from an RFID TAG (5); said processing means being configured to enable the reading of said protected health datum in relation to a validation of said user code.

10. A reader according to claim 9, wherein said processing unit (21) is configured so that said reader (2) is recognized as a HUB for removable storage units when connected to a PC and comprises at least a storage area (27) thereof in which at least one application (31) is stored, able to be automatically run from the PC (Autorun) when the reader is connected to a PC.
